# EUROPEAN PATENT SPECIFICATION

(11) **EP 3 302 592 B1**
(45) Date of publication and mention of the grant of the patent: **09.01.2019**
(21) Application number: 15753787.9
(22) Date of filing: 27.05.2015
(51) Int. Cl.: A61L 27/34, C08L 83/04

(54) **ANTIMICROBIAL COATING COMPOSITION FOR DENTAL IMPLANTS**
ANTIMIKROBIELLE BESCHICHTUNGSZUSAMMENSETZUNG FÜR ZAHNIMPLANTATE
COMPOSITION DE REVÊTEMENT ANTIMICROBIENNE POUR IMPLANTS DENTAIRES

(43) Date of publication of application: 11.04.2018
(73) Proprietor: Edierre Implant System S.p.A., 16146 Genova (IT)
(72) Inventor: BIGNOZZI, Carlo Alberto, 44121 Ferrara (IT); CARINCI, Francesco, 40124 Bologna (IT)
(74) Representative: Zambardino, Umberto
(86) International application number: PCT/IT2015/000142
(87) International publication number: WO 2016/189556

(56) References cited:
- WO-A2-2013/131994
- US-A1- 2014 288 592
- VERRAEDT E ET AL: "Controlled release of chlorhexidine from amorphous microporous silica", JOURNAL OF CONTROLLED RELEASE, ELSEVIER, AMSTERDAM, NL, vol. 142, no. 1, 25 February 2010 (2010-02-25), pages 47-52, XP026892473, ISSN: 0168-3659, DOI: 10.1016/J.JCONREL.2009.09.022 [retrieved on 2009-10-03]

## Description

The present invention generally refers to an antimicrobic pharmaceutical composition comprising polysiloxane oligomers functionalized with antimicrobial components. Said composition is suitable for the coating of medical implants, particularly dental implants made of titanium and/ or its alloys.

### Background

Peri-implant infections are generally classified as peri-implant mucositis and peri-implantitis. Peri-implant mucositis is defined as a reversible inflammatory reaction in the soft tissues surrounding an implant. Symptoms and clinical signs of peri-implant infections are many: progressive increase in pocket depht, suppurating exudate from peri-implant space, bleeding on probing, gingival inflammation of the surrounding mucosa and loss of supporting bone radiographically detectable. Peri-implantitis is defined as infectious disease that causes an inflammatory process in the soft and hard tissues surrounding an osseointegrated implant, leading to the loss of supporting bone, and finally to the implant loss (for a general reference, see Sanz M, Chapple IL. J Clin Periodontol 2012; 39 (Suppl. 12):202-206). It is well understood that peri-implantitis occurs due to the presence of pathogenic micro-organisms colonizing the surrounding implant area (see, for instance, Quirynen M, Voliquids R, Peeters W, van Steenberghe D, Naert I, Haffajee A. Clin Oral Implants Res 2006;17:25-37). Also, one of the main cause of peri-implantitis consists in the passage of pathogenic bacteria in the abutment-implant gap. In this respect, Quirynen et al. demonstrated bacterial penetration between the components of the Brånemark implant system (Quirynen M, De Soete M, van Steenberghe D. Clin Oral Implants Res 2002;13:1-19). In practice, the inner spaces were easily colonized and bacteria may leak out from these spaces through the implant-abutment interface into the peri-implant area.

Many studies have shown that bacterial species of periodontal disease are very similar to those that cause peri-implantitis (Leonhardt A, Adolfsson B, Lekholm U, Wikstrom M, Dahlen G. Clin Oral Implants Res 1993;4:113-120. Heitz-Mayfield LJ, Lang NP. Periodontol 2000 2010;53:167-181) for which it is clear that blocking the passage of bacteria in the peri-implant space is essential to prevent peri-implantitis. In order to avoid or to limit the above situation, the formation of a biofilm around the implant may play a fundamental role in the onset of peri-implantitis (see, for instance, Augthun M, Conrads G. Int J Oral Maxillofac Implants 1997;12:106-112).

In this respect, the potential benefits of local antimicrobial layer binding the external and internal of implant surface, with a long lasting effect, may dramatically reduce the onset of perimplantitis and at the same time is free from adverse effects. Local delivery of antimicrobial components into the implant-abutment gap, in fact, could achieve a greater concentration of drug locally, proving bactericidal for most periopathogens, and at the same time, exhibiting negligible impact on the microflora residing in other parts of the body. However, the drug local delivery system should have a long lasting action, as well as a resistance to the physiological conditions of the interest site, such as pH and the like.

Verraedt E. et al. "Controlled release of chlorhexidine from amorphous microporous silica" Journal of Controlled Release, Elsevier, Amsterdam, NL, vol. 142 no. 1, 25 February 2010 discloses a system for the controlled release of chlorhexidine from amorphous porous silica. The silica can be coated onto a substrate such as a dental implant.

US 2014/288592 discloses a composition comprising a polysiloxane and a antimicrobial agent. A polysiloxane compound is coated onto a surface which can be a medical device. The polysiloxane coating is further functionalized with a biomolecule such as a antimicrobial agent.

WO 2013/131994 discloses a coating for dental implants comprising a polysiloxane and a bioactive agent.

From the above it follows that still remains the problem in the art of how to find out a pharmaceutical composition which can be used as antimicrobial agent, particularly in dental field as agent for the treatment of periodontal disease, which is stable and can release the active ingredient in the site of interest, in a save and prolonged manner.

The applicant has surprisingly found that when polysiloxane oligomers of general formula (I) are admixed with at least one antimicrobial agent, the resulting pharmaceutical composition can be used as coating agent for dental implant, thus exerting a function of antimicrobial agent directly on the external surface of the implant (in contact with the bone) as well as at the implant-abutment junction, thus reducing the rejection commonly attributable to inflammatory processes, and also promoting the implant osteointegration.

Advantageously, the composition can be applied to the implant surface by means of simple procedures such as immersion or spray coating, followed by rinsing and drying.

### Summary of the invention

In one aspect the invention refers to a pharmaceutical, preferably hydro-alcoholic, composition comprising a polysiloxane oligomer, or a mixture thereof, of general formula (I) and at least one antimicrobic agent: 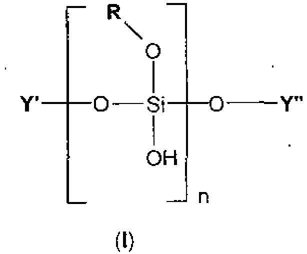 wherein:
- **R**: is independently selected from: an optionally substituted linear or branched C₁-C₁₀ alkyl, alkoxy, or alkeny group;
- **Y'** and **Y"**: are each independently selected from: an optionally substituted linear or branched C₁-C₁₀ alkyl, alkoxy, or alkeny group, a hydrogen atom (H), or a metal oxide derivative of formula: 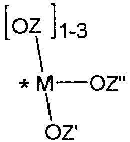
wherein M is an atom selected from: titanium(IV), zirconium(IV), vanadium(IV), molybdenum(V), tungsten(V) and tungsten(VI);
Z, is an optionally substituted linear or branched C₁-C₁₀ alkyl, alkenyl or alkoxy group,
Z' and Z" represent each independently an optionally substituted linear or branched C₁-C₁₀ alkyl, alkenyl or alkoxy group or a hydrogen atom (H),
the symbol * represents the site of connection with the terminal oxygen atom of the general formula (I), and
- **n**: is an integer comprised from 1 to 10, preferably from 3 to 5.

In a second aspect, the invention refers to a process for the preparation of said composition comprising the steps of:
- acid hydrolysis of a tetra C₁-C₁₀ alkyl silicate in the presence of an alcoholic media, and optionally in the presence of a metal tetra alkoxide derivative, to give the polysiloxane oligomers of formula (I), and
- admixture with at least one antimicrobial agent to give the present composition comprising the polysiloxane oligomers of formula (I) functionalized with said antimicrobial agent.

In a further aspect, the invention refers to the present pharmaceutical composition for use as a medicament, in particular as antimicrobial agent, more preferably in the dental field, even more preferably in the treatment of periodontal diseases, such as peri-implant infections, peri-implant mucositis and peri-implantitis.

In a still additional aspect, the invention relates to a medical implant, preferably a dental implant, made of titanium and/or its alloys, coated with said pharmaceutical composition.

Finally, the invention also refers to a process for the preparation of the present coated implant comprising the contact of a medical implant with the composition of the invention, preferably in the form of a hydro-alcoholic solution, and subsequent drying of the thus obtained coated implant.

### Description of drawings

Figure 1: mass spectrum of the polysiloxane oligomers of the invention obtained according to Scheme 1.
Figure 2: mass spectrum of the polysiloxane oligomers of the invention, after addition of titanium-tetraisopropoxide, as per Scheme 2.
Figure 3: general representation of the functionalized polysiloxane oligomers of the invention according to the present composition, wherein the antimicrobial agent is chlorhexidine digluconate, aq solution.
Figures 4a-4b: schematic representation of hydrogen bonds and covalent interactions between the pharmaceutical composition of the invention and the surface of a titanium dental implant.
Figure 5: development in Agar at 36° C for 24 h of titanium dental implants coated with the present composition according to Example 2, after the desorption experiments in water (left) and 0.6% NaCl (right), contaminated with 10⁹ UFC/ml as indicated in the text.
Figures 6a-c: development in Agar at 36° C for 24 h of the coated implants of Example 5.

### Detailed description of the invention

Unless otherwise provided, the symbol * represents the site of connection of the interested atom with the rest of the molecule.

The "term linear or branched C₁-C₁₀ alkyl or alkenyl group", refers to a group comprising from 1 to 10 carbon atom, linear or branched, satured or unsatured, such as, for example: methyl, ethyl, ethylene, propyl, propylene, isopropyl, isopropylen, butyl, butylen, ter-butyl and the like.

The term "lower alkyl group" refers to a C₁-C₆ linear or branched alkylic group having from 1 to 6 carbon atom.

The term "alkoxy group" refers to a linear or branched C₁-C₁₀ alkyl group substituted with or linked to at least one oxygen atom -O-.

The term "lower alcohol" refers to an alcohol moiety of general formula R-OH, wherein the moiety R is a lower alkyl group as above defined.

Unless otherwise provided, the term % w/w means the amount of the component with respect to the total weight of the composition.

The term "functionalized polysiloxane oligomers of formula (I)" refers to one or more polysiloxane oligomers of formula (I), obtained after interaction of the same with at least one antimicrobial agent. By that, the oligomers of formula (I) are connected by the alkyl R groups, to the selected antimicrobial agent, e.g. by way of ionic or dipole-dipole interaction, as herein set forth in more details.

The present composition is thus characterized by comprising a series of polysiloxane oligomers of formula (I), functionalized with at least one antimicrobial agent. Of note, the present composition is stable over the time, and is able to bind the surface of medical implants, such as titanium dental implants, thus trapping the antimicrobial agent over the surface of the thus coated implant. This allows the formation of an antimicrobic coating system (bound to the surface of the titanium implant) which can release very slowly the antimicrobial component over the time, directly to the region surrounding the implant, thus avoiding bacterial infections, which are considered the primary etiological factor for peri-implantitis. The present composition is also useful in favoring the osteointegration of the thus coated implant.

In particular, the polysiloxane oligomers of general formula (I) can act as a pharmaceutical carrier linking together the surface of the implant with the selected antimicrobial agent, or mixture of agents. In this respect, in fact, it has to be noted that the present composition, comprising functionalized polysiloxanes of formula (I), can interact with the surface of the implant by way of ionic interaction (such as van der Waals and the like) or by way of covalent bindings, depending on the chemical structure of the polysiloxane oligomers mixture as below further explained.

As indicated in the general formula (I), the chemical structure of the polysiloxane oligomers of the invention shows a structural portion represented by the alkyl groups R, which is able to interact with the antimicrobial agent of choice, thus providing a stable connection with this latter. On the other hand side, the present polysiloxane oligomers also comprise a structural portion, represented by the free hydroxyl groups bonded to the silane atoms, responsible for the connection with the surface of the medical implant. This latter is preferably made of titanium and the interaction with the functionalized polysiloxane oligomers of formula (1) thus results particularly effective and advantageous. Surprisingly, a medical implant made *e.g.* of titanium oxide can interact with the oligomers of formula (I), functionalized with an antimicrobial agent according to the present composition, thus exerting a long lasting antimicrobial action, as herein described in details. Advantageously by that, the functionalized oligomers of the composition of the invention can behave like a pharmaceutical carrier, in order to connect the antimicrobial agent of choice with the medical implant, by coating of its surface.

The polysiloxane oligomers of general formula (I) can be prepared by acidic hydrolysis of a corresponding tetra C₁-C₁₀-, preferably C₁-C₆-alkyl silicate, in the presence of a suitable alcoholic media, such as a lower alcohol, (i.e. having from 1 to 6 carbon atom), or a mixture of two or more lower alcohols, thus generating after a few hours a mixture of polysiloxane oligomers of general formula (I), characterized by a -[O-Si]-repeating unit, and by different substituents bound to the Si atom, which include R alkyl organic moieties, and free hydroxyl functionalities (-OH).

"Acidic hydrolysis" means that the reaction is carried out in the pH range 0-5, preferably in the pH range 1-4, by regulation of the pH upon addition of a proper amount of a strong inorganic or organic acid, such as, for example, perchloric or nitric acid.

A particularly preferred tetra C₁-C₆-alkyl silicate is the tetraethyl orthosilicate (usually abbreviated with "TEOS"; CAS registry Number: 78-10-4).

The alcoholic media is preferably a mixture of two or more alcohols, even more preferably a mixture of 2-propanol and 3-methoxy-3-methyl-1-butanol, or a mixture of 2-propanol- 3-methoxy-3-methyl-1-butanol and heptanol, or a mixture of 2-propanol-3-methoxy-3-methyl-1-butanol and octanol.

The alcoholic media comprises the selected alcohols, preferably isopropanol and 3-methoxy-3-methyl-1-butanol, in a ratio from 1:2 up to 1:10 preferably from 1:5 to 1:8. Advantageously, the alcoholic media allows to stabilize the solutions of the polysiloxane oligomers for prolonged period of times, even extending up to 12 months.

Thus, in a preferred embodiment, the tetra C₁-C₆-alkyl silicate is subjected to acidic hydrolysis, as above indicated, to give mixture of oligomers of formula (I), according to the following Scheme 1: 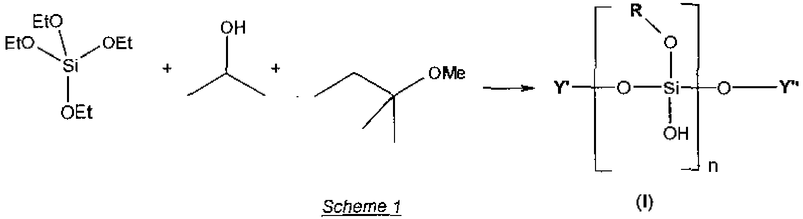 wherein:
R is a residue independently selected from: 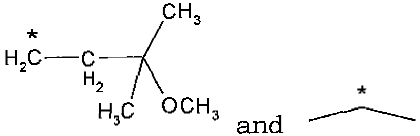
Y" is ethyl or methyl;
Y' is independently selected from hydrogen or a residue of formula: 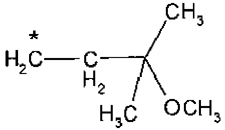
n is as above defined.

As it can be understood, since the fuctionalization of the silane atom occurs randomly, and since the oligomer reaction can lead to different numbers of repeating units -[O-Si]- (*i.e.* different n values in formula (I)), the chemical composition of the polysiloxane oligomers of formula (I) can be very complex.

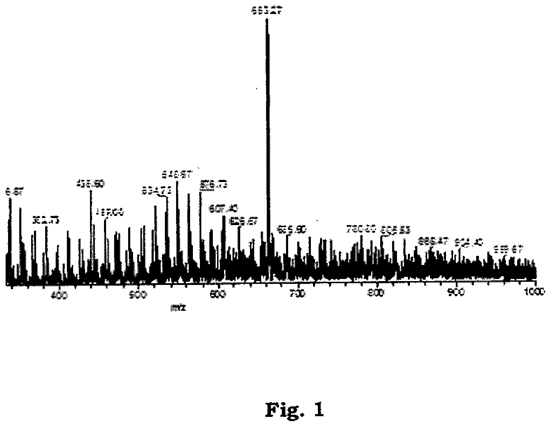

As indicated in Figure 1, the mass spectra of the polysiloxane oligomers shows the ionic peak with m/z = 663.33 (m = mass; z = charge) which can be best fitted to the following preferred formulae (**Ia** e **Ib**): 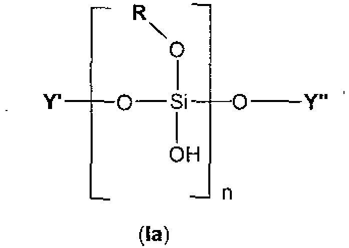 wherein:
R and Y' are: 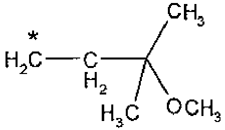
Y" is ethyl; and
n = 3,
and 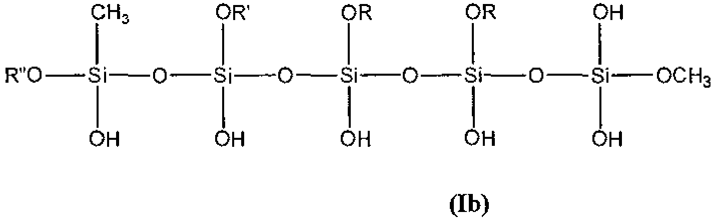 wherein:
R= 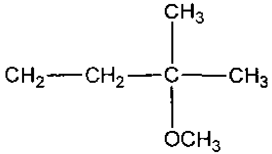
R'= 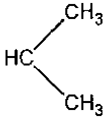
R"= H₂C-CH₃

In a still preferred embodiment of the invention, the acidic hydrolysis of the selected tetra alkoxy silane as above described is carried out also in the presence of a metal tetra alkoxide derivative, compatible with the hydroxyl groups. The term "compatible with the hydroxyl groups" means that said metal alkoxide can link the terminal siloxane moiety, thus being useful, in particular, to control the number of silane repeating units (*i.e.* n), and thus enabling the preparation of polysiloxanes of general formula (I) having a more defined chemical structure. Advantageously, the addition of said kind of metal tetra alkoxide can lead to a simplified distribution of components, also providing a remarkable stability of the functionalized oligomers of the invention, even after several months. Preferably, it is used a titanium tetra alkyloxide, more preferably a titanium tetra C₁-C₆ alkyloxide. In an embodiment, the titanium tetra alkyloxide is selected from: titanium(IV) tetraisopropoxide (CAS Registry Number: 546-68-9), titanium(IV) butoxide (CAS Registry Number: 5593-70-4), titanium(IV) tert-butoxide (CAS Registry Number: 3087-39-6), and titanium(IV) ethoxide (CAS Registry Number: 3087-36-3). Ugually preferred is a tetra alkoxide selected from: zirconium (IV) n-propoxide (CAS Registry Number: 23519-77-9), vanadium(V) triisopropoxide (CAS Registry Number: 5588-84-1), molibdenum(V) isopropoxide (CAS Registry Number: 209733-38-0); tungsten(V) etoxide (CAS Registry Number: 26143-11-3); and tungsten(VI) isopropoxide (CAS Registry Number: 52321-90-1) In a particularly preferred embodiment, said alkoxide is the titanium-tetraisopropoxide (Ti[OCH(CH₃)₂]₄ (CAS Registry Number: 546-68-9).

As will be understood, the metal M in formula (I) will present a number of -OZ residue ranging from 1 to 3 (and indicated as -[OZ]₁₋₃) depending on the oxidation number of the metal atom of the selected metal tetra oxide, as above indicated.

In any case, said selected metal oxide, preferably titanium-tetraisopropoxide, is used in amount comprised from 1% w/w to 10% w/w, being 1% w/w to 4% w/w particularly preferred.

Thus in an embodiment of the invention, Y' and Y" are each independently selected from: an optionally substituted linear or branched C₁-C₁₀ alkyl, alkoxy, or alkeny group, a hydrogen atom (H), or a group of formula: 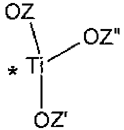 wherein Z, Z' and Z" are as above defined.

In any case, and according to a preferred embodiment, the metal tetra oxide of choice is admixed with a suitable lower alcohol, and then added to the formerly indicated mixture of alkyl silicate and alcoholic media, and allowed to react for a proper frame of time. Even more preferably, the metal tetra alkoxide of choice is solved in 3-methoxy-3-methyl-1-butanol, and then added to the polysiloxane product, the latter preferably obtained by the acid hydrolysis of TEOS, in the presence of 2 propanol, and 3-methoxy-3-methyl-1-butanol as above described.

In this case, the process for the preparation of the oligomers of formula (I) wherein Y is a titanium residue can be schematized in the following Scheme 2: 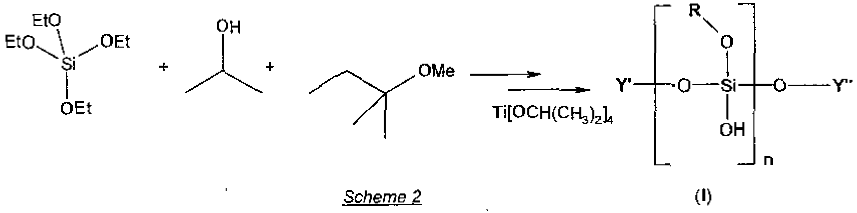 wherein:
R is a residue independently selected from: 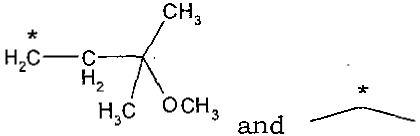
Y' is group of formula: 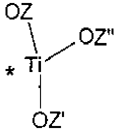
wherein:
Z and Z' are as above defined, preferably an isopropyl residues of formula: 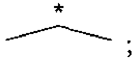
Z" is as above defined, preferably hydrogen (H),
the symbol * represents the site of connection to the rest of the molecule; Y" is methyl (-CH₃) or ethyl (-CH₂CH₃); and
n is as above defined.

The addition of the titanium tetra alkoxide is carried out at room temperature (intended as comprised from about 15-40° C), for a frame of time of about 2-3 hours.

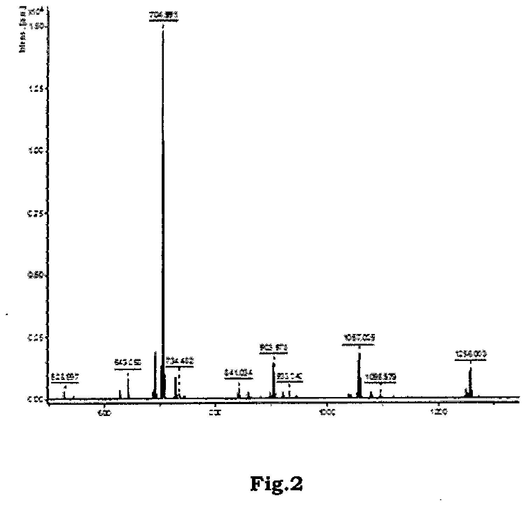

In figure 2 it is represented a MS spectra, of a mixture of oligomers (I) obtained in the presence of titanium-tetraisopropoxide (Ti[OCH(CH₃)₂]₄), according to Scheme 2. It is evident the presence of a main compound having an ionic peak with m/z = 704.99, best fitting with the following preferred formula (**Ic**):

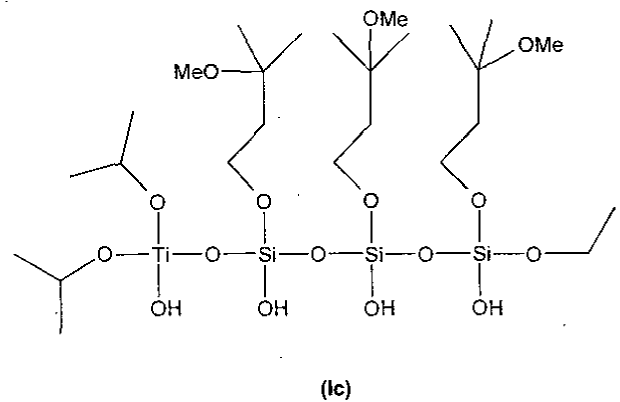

Thus, it follows that according to a preferred embodiment, in the polysiloxane oligomers of formula (I), R is independently a linear or branched C₁-C₆ alkyl and/or alkoxy group. Even more preferably R is independently a 2-isopropyl residue (-CH(CH₃)₂), or a 3-methoxy-3-methylbuthyl residue of formula:

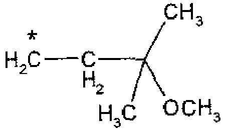

Preferably, Y' is a group of formula: 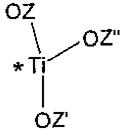 wherein Z, Z' and Z" are as above defined.

Even more preferably, Z and Z' are each independently a linear or branched C₁-C₆ alkyl group, and Z" is hydrogen (-H). In a still preferred embodiment, Z and Z' are both an isopropyl residue, and Z" is hydrogen.

In a further embodiment, Y" is a linear or branched C₁-₆ alkyl group, preferably an ethyl group.

More preferably, in the polysiloxane oligomer of formula (I), Y' is a group of formula: 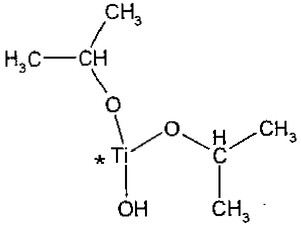 and Y" is *CH₂CH₃.

It is worth noting that, in all the cases, the backbone of the mixture of polysiloxane oligomers of general formula (I) contains silicon atoms bearing R alkyl groups, as well as free hydroxyl (-OH) groups. Said oligomers can then be properly functionalized with a suitable antimicrobial agent or mixture of agents, thus providing the pharmaceutical composition of the invention.

Surprisingly, the polysiloxane oligomers of general formula (I) are able to interact with at least one proper antimicrobial agent, being this latter preferably in the form of an aqueous media. By stirring, an interaction between siloxane and antimicrobial agent occurs, thus forming the present pharmaceutical composition comprising the polysiloxane oligomers (I) functionalized with the selected antimicrobial agent and ready to be used in particular for the coating of a medical device.

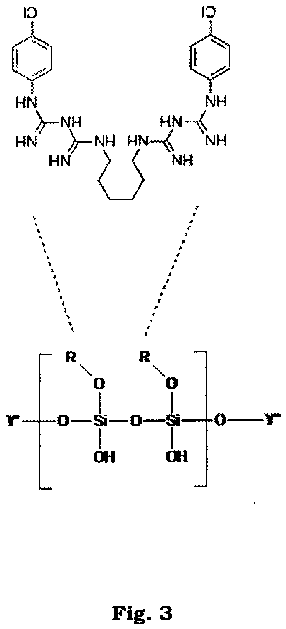

Said interaction allows the connection of the oligomers (I) with the selected antimicrobial agent, thus forming the corresponding functionalized polysiloxane oligomers (I), as generally represented in Figure 3. The R alkyl groups bound to the silicon atom, in fact, have the ability to trap specifically an antimicrobial species, typically by dipole-dipole or van der Waals interactions.

Thus, in the present composition, preferred antimicrobial agents are selected from at least one pharmaceutical acceptable salt of: chlorhexidine, didecyl-dimethyl-ammonium, and benzalkonium ions. Preferably, said pharmaceutical acceptable salts are digluconate, acetate, chloride and the like.

Preferably, the antimicrobial agent of the present composition is chlorhexidine digluconate.

The selected antimicrobial salt is preferably added to the oligomers (I) in the form of an aqueous media. Suitable aqueous media can be water for injection, pure water, distilled water, ultrapure water and the like, being ultrapure water preferred.

According to an embodiment of the invention, the antimicrobial agent is a mixture of a chlorhexidine salt, preferably digluconate, with at least one additional antimicrobial agent, being a benzalkonium salt, preferably chloride, particularly preferred. Also in this case, the two antimicrobial agents can be added to the oligomers (I) in form of an aqueous media as above reported.

In one embodiment, the antimicrobial agent (or a mixture thereof) is used in the form of an aqueous solution, preferably having a concentration comprised from about 5% w/w to 20% w/w preferably from 10% w/w to 20% w/w. The antimicrobial agent is added to the polysiloxane oligomers (I) in a total amount comprised from 0.5% w/w to 5% w/w, being 1% w/w to 3% w/w preferred. "Total amount" means the amount of a single agent, or in case of more than one agent, the final amount of the combination of the selected antimicrobial agents. Applicant has in fact noted that in this range of % w/w it is possible not only to obtain a reliable and long lasting antimicrobial action, but it is also possible to combine one or more antimicrobial agent, as for instance indicated in the herein below Experimental part, thus enhancing the antimicrobial activity of the present composition in a safe and reliable way. By that, and according to a preferred embodiment, the present composition comprises a mixture of 0.5-1.5 % w/w of chlorhexidine digluconate in admixture with 0.5-1.5 % w/w of benzalkonium chloride.

Thus, in a particularly preferred embodiment, the present pharmaceutical composition, comprising the polysiloxane oligomers of general formula (I) and at least one antimicrobial agent, is in the form of a hydro-alcoholic media, preferably in the form of a hydro-alcoholic solution. Unless otherwise provided, the term "solution" means that the hydro-alcoholic media is substantially free of residues or precipitates that indeed characterized a suspension or an heterogenic composition. Thus, the aqueous hydro-alcoholic physical form of the composition is particularly useful not only because the easiness of its application in the coating of a dental implant, but also because by drying the hydro-alcoholic media, it is possible to stably link the mixture of the functionalized oligomers (I) with the surface of the implant, as below described in more details.

As above mentioned, the interaction of the oligomers of general formula (I) with the selected antimicrobial agent (or mixture thereof) can occur by means of weak interactions such as van der Waals, dipole-dipole interaction and the like. By way of example, in the case of chlorhexidine, the functionalization of the oligomers (I) can be a consequence of the structure of the bis-biguanide backbone of the chlorhexidine, which can interact with the R alkyl groups at the silicon atoms, via Van der Waals forces as schematically represented in Figure 3. Surprisingly, said kind of interactions are capable of competing with chlorhexidine desorption from an implant coated with the present composition when in contact with an aqueous solutions, such as the saliva in case of dental implants. In other words, the interaction between the oligomers (I) and the chlorhexidine are such that after deposition of the pharmaceutical composition of the invention on a medical implant, it is possible to obtain a gradual desorption of the antimicrobial agent(s) (e.g. chlorhexidine) over the time, thus exerting a convenient and active antimicrobial action. This is of upmost relevance since by using the present pharmaceutical composition it is possible to preserve the patient from the formation of infections or inflammatory related diseases, especially in case of dental implants, i.*e*. in conditions where the proliferation of microbes is particularly damaging and difficult to control. This fact is demonstrated by a series of tests carried out by the Applicant, where some of them are included in the present Experimental part. In practice, different parts of a titanium dental implant, such as the dental implant itself, the abutments, the mounters, the tap screws and the connection screws, all made in titanium or titanium dental alloys, where contacted with a solution (abbreviated as PXT) obtained by acidic hydrolysis of TEOS in the presence of a mixture of 2-propanol and 3-methoxy-3-methyl-1-butanol in a weight ratio of 1:7, mixed 1:1 with a 3-4% w/w titanium isopropoxide solution in 3-methoxy-3-methyl-1-butanol and admixed with chlorhexidine digluconate 1% w/w. The contact can occur via immersion or even by spray. In any case, after drying, in oven, at a temperature of about 40-80° C for 30-60 min, the thus coated implant is immersed in 20 ml of distilled water or 20 ml of a physiological solution containing 0.6% NaCl and kept at 36° C for several days.

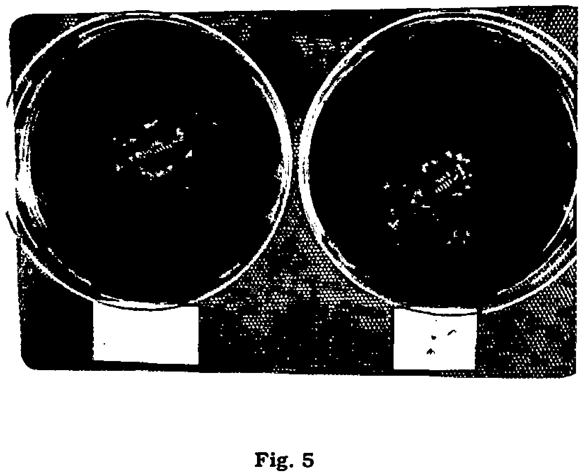

In this respect, the electronic absorption spectra monitored on physiological and pure aqueous solutions in contact with the PXT treated titanium implants shows that the increase of the optical absorption intensity in the UV region, as a function of time, is consistent with a very slow release of chlorhexidine, which exhibit π-π* electronic transitions in the 200-350 nm range. Tests of antimicrobial activity, on the different components, demonstrated, despite chlorhexidine desorption, an extraordinary activity, testifying retention of chlorhexidine in the polysiloxane layer. Figure 5 shows that two implants kept in contact respectively with water or with a physiological solution, rinsed in water, dried and put in contact with 0.1 ml suspension of a microbic pool constituted by: Staphilococcus aureus, ATCC 6538; Escherichia coli, ATCC 10536; Pseudomonas aeruginosa, ATCC 15442; Enterococcus hirae, ATCC 10541 and Candida albicans, ATCC 10231, at the concentration level of 10⁹ ufc/ml show very clear inhibition rings due to the presence of chlorhexidine, retained at the surface of the implant.

It should be noted that, in practice, the amount of the present antimicrobial pharmaceutical composition which can be in contact with the implanted implant, or at the implant-abutment junction, can vary from about 50 to 20 microliters. As a consequence, scaling the desorption of chlorhexidine linearly, it can be safely concluded that the antimicrobial effect should last even for several tens of years.

Advantageously, the local delivery of the pharmaceutical composition of the invention directly into the implant-abutment gap, achieves a greater locally concentration of the antimicrobic agent(s), proving a bactericidal action for most of periopathogens, and at the same time, exhibiting negligible impact on the microflora residing in other parts of the body.

As a skilled in the art will understand, the present composition may also comprise additional pharmaceutical acceptable additives, commonly use, for example, in the dental field or as used in the art for preparation of antimicrobial compositions in general.

In a further aspect, the invention refers to a medical implant, coated with the present pharmaceutical composition.

The coated implant can be obtained by a process that comprises the steps of:
- contacting the medical implant with the present composition, preferably in the form of a hydro-alcoholic media, thus coating the surface of said implant, and
- drying the thus coated implant.

In this respect, the contact step can be performed by immersion, spraying or similar, of the implant. The liquid physical form of the present pharmaceutical composition, in fact, is particularly suitable for these kind of operation, thus allowing a very easy and reliable method of preparation of a coated medical implant. In a preferred embodiment, the implant is immersed in the composition of the invention, this latter in the form of a hydro-alcoholic solution.

In the preparation of the coated medical implant of the invention, it has to be noted that after the contact between the implant and the present composition, the drying step is responsible for the evaporation of the water and the alcohol content, thus enabling the linking of the free hydroxyl groups of the general formula (I) with the implant surface, namely the metal oxide, preferably TiO₂. In this respect the drying temperature is comprised between about 40° C and 80° C being a temperature comprised between about 50° C and 65° C preferred. Advantageously, the drying step can be carried out at atmospheric pressure, using apparatus known to the skilled in the art, such as, ovens, air flows or the like.

It is worth to note that, in principle, any kind of material or substrate which is acceptable from a physiological point of view and which can interact with the present composition comprising the functionalized polysiloxane oligomers of formula (I) can actually be coated with the present composition, and used as a medical coated antimicrobial implant. In a preferred embodiment, the invention refers to dental implant, made of, or even coated with, TiO₂ or Ti alloys, coated with the present composition.

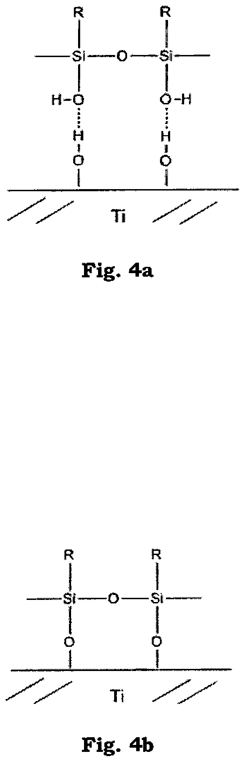

In particular, implants made of titanium can oxidize their surface in air to titanium dioxide (TiO₂), with a stochastic distribution of the two crystalline forms rutile and anatase. Surprisingly it has been found that the oxide layer at the surface, covered by functional Ti-OH groups, is able to interact very quickly with the present composition, as above described. The stability of the polysiloxane coating on the titanium surface can thus be rationalized in terms of chemical bonding, since the presence of -OH functions on the titanium oxide layer can give rise to the formation of hydrogen bonds with the siloxane moieties, as schematized in figure 4a, or even to the formation of a covalent bonds, as schematized in figure 4b.

Thus, in a still preferred embodiment, the medical implant has at least the surface made of titanium oxide and/or titanium alloys. The term "medical implant" comprises the implants, for example in forms of prosthesis, or dental implant in general, that can be used in the medical field.

In a particularly preferred embodiment, the coated implant is a dental implant, more preferably in the form of dental screw, abutments, mounters, tap screws and connection screws.

Advantageously, and as above reported, the coated implant of the invention can release very slowly the antimicrobial agent(s) to the region surrounding the implant, thus avoiding bacterial infections, which are considered the primary etiological factor for peri-implantitis. Surprisingly, the release of the antimicrobial agent(s), from the implant coated with the present composition was observed to be extremely slow, thus keeping the implanted surface of titanium protected from bacterial attack for a very long period of time, which was estimated of the order of several years.

Even further, the present coated medical implant not only can exert a high anti-infective action as above explained in details, but can also maintain or even improve the osteointegrative properties of the titanium itself, thus representing a significant advance over currently available osteointegrated biomedical prosthetic devices.

In a still additional aspect, the present invention refers to the use of the present composition comprising the polysiloxane oligomers (I) and at least one antimicrobial agent as above described as a medicament, in particular as antimicrobial agent, preferably for use in the dental field, even more preferably in the treatment of periodontal diseases and/or peri-implant infections. In an additional embodiment, the invention thus refers to the use of the present composition for the preparation of a medicament, preferably for the treatment of peri-implant infections.

The invention also refers to the use of the present composition as a coating agent for medical implant. Preferably in this respect, the present composition is for use as coating agent for dental implant, even more preferably for dental implants having at least the surface made of titanium and/or its alloys.

The invention will be now described by the following not limiting experimental part.

### EXPERIMENTAL PART

### Example 1: preparation of a hydro-alcoholic solution comprising the polysiloxane oligomers of formula (I) of the invention, functionalized with chlorhexidine (PXT)

Tetraethoxysilane was hydrolyzed for 2-8 h at room temperature in a solution containing a weight ratio of 1: 7: 0.7 of 2-propanol, 3-methoxy-3-methyl-1-butanol and water, in the pH range 1-4, adjusted by addition of a strong acid, e.g. perchloric acid. To this solution, titanium(IV) tetraisopropoxide, previously dissolved in 3-methoxy-3-methyl-1-butanol, was added with stirring up to reach a Ti:Si ratio of 1:4. The solution was kept stirring for additional for 2 h and then chlorexidine digluconate was added to a final concentration of 1 or 2%.

### Example 2: preparation of a dental implant of the invention, coated with the composition of the invention as prepared in the Example 1 (PXT)

A dental implant was immersed in the hydro-alcoholic solution of the Example 1.

After 10 min, the implant is pull out from the PXT solution and dried at temperature of 65°C, for 1 hour, by means of an oven.

The thus coated implant is than tested as antimicrobic agent in the following examples.

### Example 3: Retention/ desorption test of the coated implant of the Example 2.

The electronic absorption spectra was monitored on physiological and pure aqueous solutions in contact with the PXT coated titanium implants of Example 2. The increase of the optical absorption intensity in the UV region, as a function of time, is consistent with chlorhexidine release, which exhibit π-π* electronic transitions in the 200-350 nm range. Test of antimicrobial activity, on the different components, demonstrated an extraordinary activity, testifying retention of chlorhexidine in the polysiloxane layer.

### Example 4a-b: antimicrobial test of the coated implant of Example 2.

### Example 4a.

Figure 5 shows that the two implants coated according to Example 2 and kept in contact respectively with water or with a physiological solution, rinsed in water, dried and that put in contact with 0.1 ml suspension of a microbial pool constituted by: Staphilococcus aureus, ATCC 6538; Escherichia coli, ATCC 10536; Pseudomonas aeruginosa, ATCC 15442; Enterococcus hirae, ATCC 10541 and Candida albicans, ATCC 10231, at the concentration level of 10⁹ ufc/ml showed very clear inhibition rings due to the presence of chlorhexidine, retained at the surface of the implant.

### Example 4b.

Additional microbiological tests were performed in order to evaluate the efficacy of the composition of the invention (PXT) as antimicrobial coating in killing bacteria infiltrated between implant and abutment. Testing were performed on the inside of PXT treated and untreated implants, which were externally contaminated with P. gingivalis and T. forsythia, considered the first pathogens involved in the clinical destruction of peri-implant bone and in the local development of peri-implantitis. It was observed that the median percentage of bacterial passage toward the implant junction was comparable in treated and untreated implants, but evaluation of viability at time points of 24, 48 and 72 hours revealed that no bacteria were alive in the treated implants while bacterial growth was observed for the untreated ones.

### Example 5: antimicrobial activity of an implant coated with the composition of the invention and sterilized by Gamma radiation.

Before application, the dental implants typically undergo sterilization, which is normally performed with gamma irradiation. A series of tests were conducted in order to verify if gamma radiation was interfering with the efficacy of the antimicrobial treatment on implants previously coated with an antimicrobial composition of the invention containing:
- chlorhexidine digluconate at 1% or 2% (composition 1 or 2); and
- a mixture of chlorhexidine digluconate at 1% and benzalkonium chloride at 1% (composition 3).

Implant components were treated with the procedure of Example 2, and then irradiated with gamma rays.

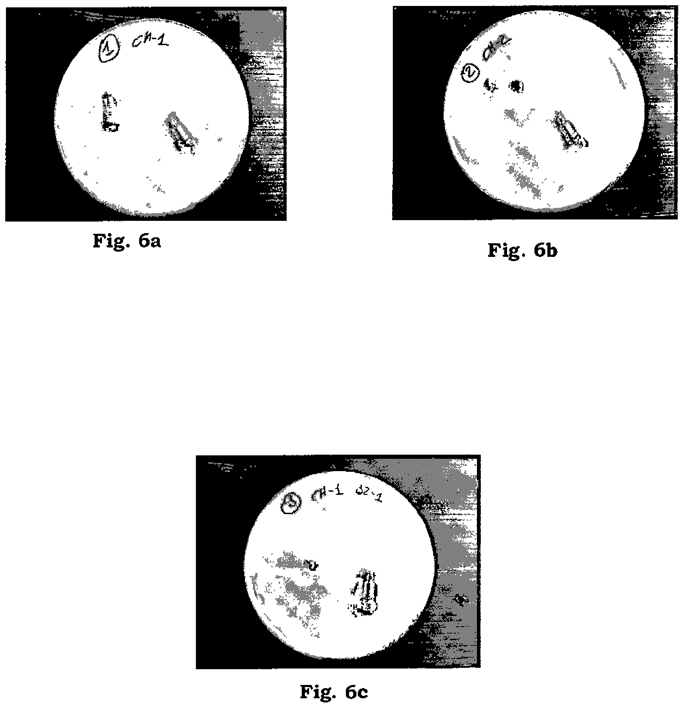

The component of the dental implants were then put in contact with 0.1 ml of a microbial pool constituted by: Staphilococcus aureus, ATCC 6538; Escherichia coli, ATCC 10536; Pseudomonas aeruginosa, ATCC 15442; Enterococcus hirae, ATCC 10541 and Candida albicans, ATCC 10231, at the concentration level of 10⁹ ufc/ml. Figures 6a-c clearly show that, after 24 h in Agar at 36° C, all dental implants components treated with the compositions 1, 2 or 3, show clear inhibition rings testifying the retention of the microbial activity of the coatings, even after gamma irradiation.

## Claims

1. A pharmaceutical composition comprising polysiloxane oligomers of general formula (I) and at least one antimicrobic agent: 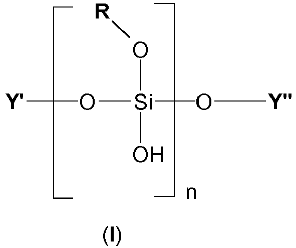 wherein:
**R** are each independently selected from: an optionally substituted linear or branched C₁-C₁₀ alkyl, alkoxy, or alkeny group;
**Y'** and **Y"** are each independently selected from: an optionally substituted linear or branched C₁-C₁₀ alkyl, alkoxy, or alkeny group, a hydrogen atom (H), or a metal oxide residue of formula: 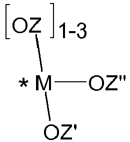
wherein M is an atom selected from: titanium(IV), zirconium(IV), vanadium(IV), molybdenum(V), tungsten(V) and tungsten(VI);
Z, is an optionally substituted linear or branched C₁-C₁₀ alkyl or alkenyl group,
Z' and Z" represent each independently an optionally substituted linear or branched C₁-C₁₀ alkyl or alkoxy group or a hydrogen atom (H),
and
the symbol * represents the site of connection with the terminal oxygen atom of the general formula (I), and
**n** is an integer comprised from 1 to 10, preferably from 3 to 5.

2. The pharmaceutical composition according to claim 1, wherein R is a residue independently selected from: 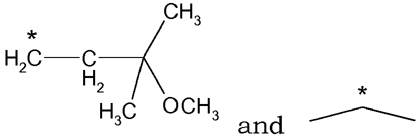 or R is independently a linear or branched C₁-C₆ alkyl and/or alkoxy group, preferably independently selected from: a 2-isopropyl residue (-CH(CH₃)₂), or a 3-methoxy-3-methylbuthyl residue of formula: 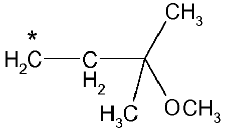 and wherein
Y" is ethyl or methyl;
Y' is independently selected from hydrogen or a residue of formula: 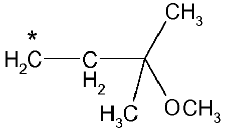
or Y' and Y" are each independently selected from: an optionally substituted linear or branched C₁-C₁₀ alkyl, alkoxy, or alkeny group, a hydrogen atom (H), or a titanium derivative of formula: 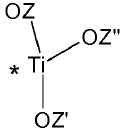
wherein Z, Z' and Z" are as defined in claim 1.
and wherein n is comprised from 3 to 5.

3. The pharmaceutical composition according to the preceding claims, wherein said Z and Z' are each independently a linear or branched C₁-C₆ alkyl group, and Z" is hydrogen or wherein said Z and Z' are both an isopropyl residue, and Z" is hydrogen.

4. The pharmaceutical composition according to the preceding claims, wherein Y" is a linear or branched C₁-₆ alkyl group, preferably a methyl or ethyl group.

5. A pharmaceutical composition according to the preceding claims, wherein:
Y' is a group of formula: 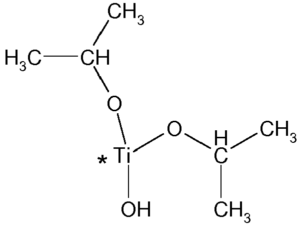
Y" is *CH₂CH₃,
R is as defined in claim 3, and
n is comprised from 3 to 5.

6. The pharmaceutical composition according to claim 1, wherein:
R and Y' are: 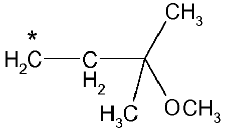
Y" is *CH₂CH₃; and
n = 3.

7. The pharmaceutical composition according to the preceding claims, wherein said antimicrobial agent is a pharmaceutical acceptable salt of: chlorhexidine, didecyldimethylammonium, benzalkonium, or mixture thereof or wherein said antimicrobial agent is a pharmaceutical acceptable salt of chlorhexidine, preferably digluconate.

8. The pharmaceutical composition according to claim 7, wherein said chlorhexidine digluconate is in admixture with at least another antimicrobial agent, preferably benzalkonium chloride.

9. The pharmaceutical composition according to the preceding claims, in form of a hydro-alcoholic media, preferably a hydro-alcoholic solution.

10. A process for the preparation of the pharmaceutical composition according to the preceding claims, comprising the steps of:
- acid hydrolysis of a tetra C₁-C₁₀ alkyl silicate in the presence of an alcoholic media, and optionally in the presence of a metal tetra alkoxide derivative, to give the polysiloxane oligomers of formula (I), and
- admixture with at least one antimicrobial agent, preferably in the form of an aqueous media, to give polysiloxane oligomers of formula (I) functionalized with said antimicrobial agent.

11. The process according to claim 10, wherein said alkyl silicate is tetraethyl orthosilicate (TEOS).

12. The process according to claims 10-11 wherein said alcoholic media comprises 2 propanol and 3-methoxy-3-methyl-1-butanol, optionally also in admixture with heptanol or octanol.

13. The process according to claims 10-12, wherein said metal tetra alkoxide derivative is selected from: a titanium tetra C₁-C₆ alkyloxide, zirconium (IV) n-propoxide, vanadium(V) triisopropoxide, molibdenum(V) isopropoxide, tungsten(V) etoxide, and tungsten(VI) isopropoxide or wherein said metal tetra alkoxide derivative is selected from: titanium(IV) tetraisopropoxide, titanium(IV) butoxide, titanium(IV) tert-butoxide, and titanium(IV) ethoxide, being the titanium-tetraisopropoxide (Ti[OCH(CH₃)₂]₄ particularly preferred.

14. The process according to claim 13 wherein said metal tetra alkoxide derivative is in admixture with at least a linear or branched C₁-C₆ alkyl alcohol which is 3-methoxy-3-methyl-1-butanol.

15. The pharmaceutical composition according to claims 1-9, for use as a medicament.

16. The pharmaceutical composition according to claim 15, for use as antimicrobial agent.

17. The pharmaceutical composition according to claims 15-16, for use as a medicament in the treatment of periodontal diseases, peri-implant infections, peri-implant mucositis and peri-implantitis.

18. Use of the composition according to claims 1-9 as a coating agent for medical implant, preferably dental implant.

19. A medical implant coated with the composition of claims 1-9.

20. The coated medical implant according to claim 19, in form of dental implant, dental screw, abutments, mounters, tap screws and connection screws.

21. The coated implant of claims 19-20, having at least its surface made of titanium and/or titanium alloys.

22. A process for the preparation of the coated medical implant of claims 19-21, comprising the steps of:
- contacting a medical implant with the present composition, preferably in the form of a hydro-alcoholic media, thus coating the surface of said implant, and
- drying the thus coated implant, preferably at a temperature comprised between 35° C and 80° C.

## Patentansprüche

1. Eine pharmazeutische Zusammensetzung enthaltend Polysiloxan-Oligomere der allgemeinen Formel (I) und mindestens einen antimikrobiellen Wirkstoff: 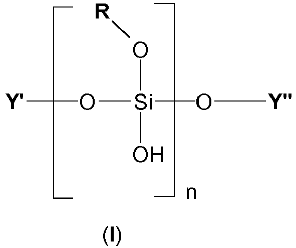 wobei:
R jeweils unabhängig ausgewählt ist aus: einer gegebenenfalls substituierten, linearen oder verzweigten C₁-C₁₀ Alkyl-, Alkoxy-, oder Alkenylgruppe;
Y' und Y" jeweils unabhängig ausgewählt sind aus: einer gegebenenfalls substituierten linearen oder verzweigten C₁-C₁₀ -Alkyl-, Alkoxy-, oder Alkenylgruppe, einem Wasserstoffatom (H) oder einem Metalloxidrest der Formel: 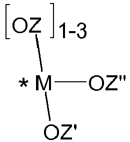
wobei M ein Atom ausgewählt aus: Titan (IV), Zirkonium (IV), Vanadium (IV), Molybdän (V), Wolfram (V) und Wolfram (VI) ist;
Z ist eine gegebenenfalls substituierte lineare oder verzweigte C₁-C₁₀ Alkyl- oder Alkenylgruppe;
Z' und Z" stehen unabhängig voneinander für eine gegebenenfalls substituierte lineare oder verzweigte C₁-C₁₀ Alkyl- oder Alkoxygruppe oder ein Wasserstoffatom (H),
und
das Symbol * stellt den Verknüpfungsort mit dem endständigen Sauerstoffatom der allgemeinen Formel (I) dar und n ist eine ganze Zahl von 1 bis 10, vorzugsweise von 3 bis 5.

2. Die pharmazeutische Zusammensetzung nach Anspruch 1, wobei R ein Rest ist, der unabhängig ausgewählt ist aus: 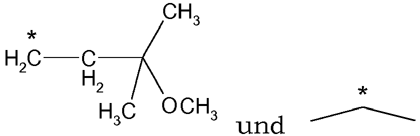 oder R unabhängig voneinander eine lineare oder verzweigte C₁- C₆ Alkyl- und/oder Alkoxygruppe ist, vorzugsweise unabhängig ausgewählt aus: einem 2-Isopropyl-Rest (-CH(CH₃)₂) oder einem 3-Methoxy-3-methylbutyl-Rest der Formel: 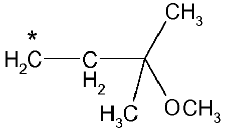 und wobei
Y" Ethyl oder Methyl ist;
Y' unabhängig ausgewählt ist aus Wasserstoff oder einem Rest der Formel: 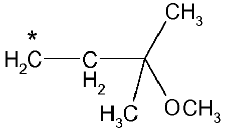 oder Y' und Y" jeweils unabhängig voneinander ausgewählt sind aus: einer gegebenenfalls substituierten linearen oder verzweigten C₁- C₁₀-Alkyl-, Alkoxy- oder Alkenylgruppe, einem Wasserstoffatom (H) oder einem Titanderivat der Formel: 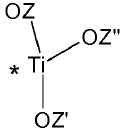 wobei Z, Z' und Z" die in Anspruch 1 angegebene Bedeutung haben und wobei n 3 bis 5 ist.

3. Die pharmazeutische Zusammensetzung nach den vorhergehenden Ansprüchen, wobei die genannten Z und Z' jeweils unabhängig voneinander eine lineare oder verzweigte C₁- C₆-Alkylgruppe sind, und Z" für Wasserstoff steht oder wobei die genannten Z und Z' beide für einen Isopropylrest stehen und Z" für Wasserstoff steht.

4. Die pharmazeutische Zusammensetzung gemäß den vorhergehenden Ansprüchen, wobei Y" eine lineare oder verzweigte C₁-C₆-Alkylgruppe, vorzugsweise eine Methyl- oder Ethylgruppe, ist.

5. Eine pharmazeutische Zusammensetzung nach den vorhergehenden Ansprüchen, wobei:
Y' eine Gruppe der Formel ist: 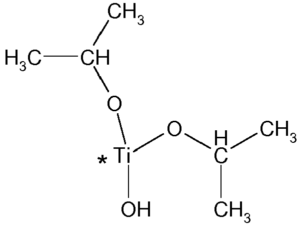
Y" ist *CH₂CH₃
R ist wie in Anspruch 3 definiert, und
n ist 3 bis 5.

6. Die pharmazeutische Zusammensetzung nach Anspruch 1, wobei:
R und Y' sind: 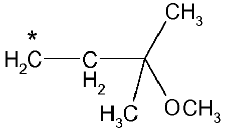
Y" ist *CH₂CH₃ und
n=3.

7. Die pharmazeutische Zusammensetzung nach den vorhergehenden Ansprüchen, wobei der genannte antimikrobielle Wirkstoff ein pharmazeutisch akzeptables Salz ist von: Chlorhexidin, Didecyldimethylammonium, Benzalkonium oder Mischung davon oder wobei es sich bei dem genannten antimikrobiellen Wirkstoff um ein pharmazeutisch akzeptables Salz von Chlorhexidin, vorzugsweise Digluconat handelt.

8. Die pharmazeutische Zusammensetzung gemäß Anspruch 7, wobei das genannte Chlorhexidindigluconat sich in einem Gemisch mit mindestens einem weiteren antimikrobiellen Wirkstoff, vorzugsweise Benzalkoniumchlorid, befindet.

9. Die pharmazeutische Zusammensetzung nach den vorhergehenden Ansprüchen in Form eines hydroalkoholischen Mediums, vorzugsweise einer hydroalkoholischen Lösung.

10. Ein Verfahren zur Herstellung der pharmazeutischen Zusammensetzung nach den vorhergehenden Ansprüchen, umfassend die Schritte:
- saure Hydrolyse eines Tetra C₁- C₁₀ Alkylsilikats in Gegenwart eines alkoholischen Mediums, und gegebenenfalls in Gegenwart eines Metalltetraalkoxid-Derivats, um die Polysiloxan-Oligomere der Formel (I) zu erhalten, und
- Zumischen mindestens eines antimikrobiellen Wirkstoffs, vorzugsweise in Form eines wässrigen Mediums, unter Erhalt von mit dem genannten antimikrobiellen Wirkstoff funktionalisierten Polysiloxan-Oligomeren der Formel (I).

11. Das Verfahren nach Anspruch 10, wobei es sich bei dem genannten Alkylsilikat um Tetraethylorthosilikat (TEOS handelt).

12. Das Verfahren nach den Ansprüchen 10 -11, wobei das genannte alkoholische Medium 2-Propanol und 3-Methoxy-3-methyl-1-butanol enthält, gegebenenfalls auch im Gemisch mit Heptanol oder Octanol.

13. Das Verfahren nach den Ansprüchen 10 -12, wobei das genannte Metall Tetraalkoxid-Derivat ausgewählt ist aus: einem Titantetra- C₁- C₆Alkyloxid, Zirkon (IV) n-propoxid, Vanadium (V) triisopropoxid, Molybdän (V) isopropoxid, Wolfram (V) ethoxid und Wolfram (VI) isopropoxid besteht, oder wobei das genannte Metalltetraalkoxid-Derivat ausgewählt ist aus: Titan (IV) Tetraisopropoxid, Titan (IV) butoxid, Titan (IV) Tert-butoxid und Titan (IV) ethoxid, wobei es sich bei das Titan-tetraisopropoxid (Ti[OCH(CH₃)₂]₄ um das besonders bevorzugte handelt.

14. Das Verfahren nach Anspruch 13, wobei das genannte Metalltetraalkoxid-Derivat in einer Zumischung mit mindestens einem linearen oder verzweigten C₁-C₆-Alkylalkohol vorliegt, der 3-Methoxy-3-methyl-1-butanol ist.

15. Die pharmazeutische Zusammensetzung gemäß den Ansprüchen 1 -9 zur Verwendung als Medikament.

16. Die pharmazeutische Zusammensetzung gemäß Anspruch 15 zur Verwendung als antimikrobielles Mittel.

17. Die pharmazeutische Zusammensetzung nach den Ansprüchen 15 -16 zur Verwendung als Medikament bei der Behandlung von Paradontose Krankheiten, periimplantären Infektionen, periimplantärer Mukositis und Periimplantitis.

18. Verwendung der Zusammensetzung nach den Ansprüchen 1 bis 9 als Beschichtungsmittel für ein medizinisches Implantat, vorzugsweise Dentalimplantat.

19. Ein medizinisches Implantat beschichtet mit der Zusammensetzung nach den Ansprüchen 1 -9.

20. Das beschichtete medizinische Implantat nach Anspruch 19 in Form eines dentalen Implantats, von Zahnschrauben, Stützpfosten, Mountern, Stichschrauben und Verbindungsschrauben.

21. Das beschichtete Implantat nach den Ansprüchen 19 - 20, bei dem zumindest seine Oberfläche aus Titan und/oder Titanlegierungen hergestellt ist.

22. Ein Verfahren zur Herstellung des beschichteten medizinischen Implantats nach den Ansprüchen 19 -21, umfassend die Schritte:
- Kontaktieren eines medizinischen Implantats mit der vorliegenden Zusammensetzung, vorzugsweise in Form eines hydroalkoholischen Mediums, wodurch die Oberfläche des genannten Implantats beschichtet wird, und
- Trocknen des so beschichteten Implantats, vorzugsweise bei einer Temperatur zwischen 35°C und 80°C.

## Revendications

1. Composition pharmaceutique comprenant des oligomères de polysiloxane de formule générale (I) et au moins un agent antimicrobien : 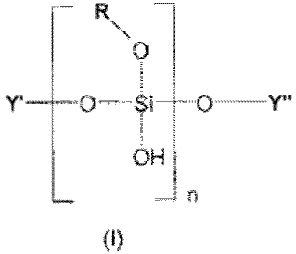 dans laquelle
R sont chacun indépendamment des autres choisis parmi un groupe alkyle, alcoxy ou alcényle en C₁-C₁₀ à chaîne linéaire ou ramifiée, éventuellement substitué ;
Y' et Y" sont chacun indépendamment de l'autre choisis parmi un groupe alkyle, alcoxy ou alcényle en C₁-C₁₀ à chaîne droite ou ramifiée, éventuellement substitué, un atome d'hydrogène (H) ou un résidu d'oxyde métallique de formule : 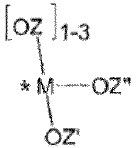
dans laquelle M est un atome choisi parmi le titane(IV), le zirconium(IV), le vanadium(IV), le molybdène(V), le tungstène(V) et le tungstène(VI) ;
Z représente un groupe alkyle ou alcényle en C₁-C₁₀ à chaîne linéaire ou ramifiée, optionnellement substitué,
Z' et Z" représentent chacun indépendamment de l'autre un groupe alkyle ou alcoxy en C₁-C₁₀ à chaîne droite ou ramifiée, en option substitué, ou un atome d'hydrogène (H),
et
le symbole * représente le site de liaison avec l'atome d'oxygène terminal de formule générale (I), et
n est un entier compris entre 1 et 10, de préférence entre 3 et 5.

2. Composition pharmaceutique selon la revendication 1, dans laquelle R est un résidu choisi d'une manière indépendante parmi 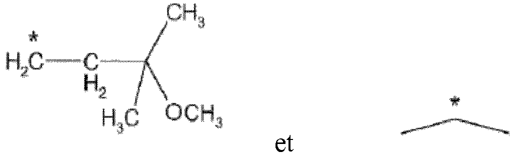 ou R est d'une manière indépendante un groupe alkyle et/ou alcoxy en C₁-C₆ à chaîne linéaire ou ramifiée, de préférence choisi d'une manière indépendante parmi un résidu 2-isopropyle (-CH(CH₃)₂), ou un résidu 3-méthoxy-3-méthylbutyle de formule : 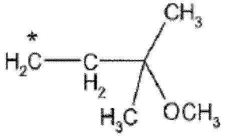 et dans laquelle
Y" est le groupe éthyle ou méthyle ;
Y' est choisi d'une manière indépendante parmi l'hydrogène ou un résidu de formule : 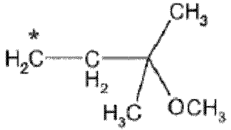
ou Y' et Y" sont choisis chacun d'une manière indépendante parmi un groupe alkyle, alcoxy ou alcényle en C₁-C₁₀ à chaîne linéaire ou ramifiée, en option substitué, un atome d'hydrogène (H) ou un dérivé du titane de formule : 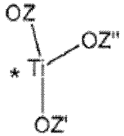
dans laquelle Z, Z' et Z" sont tels que définis dans la revendication 1, et dans laquelle n est compris entre 3 et 5.

3. Composition pharmaceutique selon les revendications précédentes, dans laquelle lesdits Z et Z' représentent chacun d'une manière indépendante un groupe alkyle en C₁-C₆ à chaîne linéaire ou ramifiée, et Z" est un atome d'hydrogène, et dans laquelle lesdits Z et Z' sont tous les deux des résidus isopropyle, et Z" est un atome d'hydrogène.

4. Composition pharmaceutique selon les revendications précédentes, dans laquelle Y" est un groupe alkyle en C₁-₆ à chaîne linéaire ou ramifiée, de préférence un groupe méthyle ou éthyle.

5. Composition pharmaceutique selon les revendications précédentes, dans laquelle
Y' est un groupe de formule : 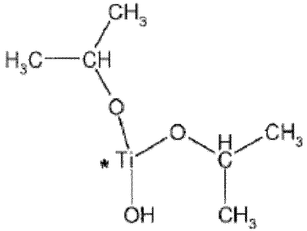
Y" est *CH₂CH₃,
R est tel que défini dans la revendication 3, et
n est compris entre 3 et 5.

6. Composition pharmaceutique selon la revendication 1, dans laquelle :
R et Y' sont : 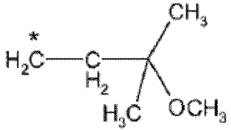
Y" est *CH₂CH₃ ; et
n = 3.

7. Composition pharmaceutique selon les revendications précédentes, dans laquelle ledit agent antimicrobien est un sel pharmaceutiquement acceptable de : chlorhexidine, didécyldiméthylammonium, benzalkonium ou d'un mélange de ceux-ci, ou dans laquelle ledit agent antimicrobien est un sel pharmaceutiquement acceptable de chlorhexidine, de préférence le digluconate.

8. Composition pharmaceutique selon la revendication 7, dans laquelle ledit digluconate de chlorhexidine est en mélange avec au moins un autre agent antimicrobien, de préférence le chlorure de benzalkonium.

9. Composition pharmaceutique selon les revendications précédentes, sous forme d'un milieu hydro-alcoolique, de préférence d'une solution hydro-alcoolique.

10. Procédé de préparation d'une composition pharmaceutique selon les revendications précédentes, comprenant les étapes suivantes :
- hydrolyse acide d'un silicate de tétra(alkyle en Ci-Cio) en présence d'un milieu alcoolique, et optionnellement en présence d'un dérivé de tétraalcoxyde métallique, pour donner les oligomères de polysiloxane de formule (I), et
- mélange avec au moins un agent antimicrobien, de préférence sous forme d'un milieu aqueux, pour donner les oligomères de polysiloxane de formule (I) fonctionnalisés avec ledit agent antimicrobien.

11. Procédé selon la revendication 10, dans lequel ledit silicate d'alkyle est l'orthosilicate de tétraéthyle (TEOS).

12. Procédé selon les revendications 10-11, dans lequel ledit milieu alcoolique comprend du 2-propanol et du 3-méthoxy-3-méthyl-1-butanol, optionnellement en mélange avec de l'heptanol ou de l'octanol.

13. Procédé selon les revendications 10-12, dans lequel ledit dérivé de tétraalcoxyde métallique est choisi parmi : un tétra(alkyle en C₁-C₆)oxyde de titane, le n-propoxyde de zirconium(IV), le triisopropoxyde de vanadium(V), l'isopropoxyde de molybdène(V), l'éthoxyde de tungstène(V) et l'isopropoxyde de tungstène(VI), ou dans lequel ledit dérivé de tétraalcoxyde métallique est choisi parmi le tétraisopropoxyde de titane(IV), le butoxyde de titane(IV), le tert-butoxyde de titane(IV) et l'éthoxyde de titane(IV), le tétraisopropoxyde de titane (Ti[OCH(CH₃)₂]₄ étant particulièrement préféré.

14. Procédé selon la revendication 13, dans lequel ledit dérivé de tétraalcoxyde métallique est en mélange avec au moins un alcool alkylique en C₁-C₆ à linéaire ou ramifiée, qui est le 3-méthoxy-3-méthyl-1-butanol.

15. Composition pharmaceutique selon les revendications 1-9, pour une utilisation en tant que médicament.

16. Composition pharmaceutique selon la revendication 15, pour une utilisation en tant qu'agent antimicrobien.

17. Composition pharmaceutique selon les revendications 15-16, pour une utilisation en tant que médicament lors du traitement de maladies parodontiques, d'infections péri-implantaires, de la mucosité péri-implantaire et la péri-implantite.

18. Utilisation de la composition selon les revendications 1-9 en tant qu'agent de revêtement pour implant médical, de préférence pour implant dentaire.

19. Implant médical revêtu de la composition selon les revendications 1-9.

20. Implant médical revêtu selon la revendication 19, sous forme d'un implant dentaire, d'une vis dentaire, de piliers, de modèles de montage, de vis auto-taraudeuses et de vis de connexion.

21. Implant revêtu selon les revendications 19-20, dont au moins la surface est fabriquée en titane et/ou en alliages de titane.

22. Procédé de préparation de l'implant médical revêtu selon les revendications 19-21, comprenant les étapes suivantes :
- à mettre en contact un implant médical avec la composition de la présente invention, de préférence sous forme d'un milieu hydro-alcoolique, de façon à revêtir la surface dudit implant, et
- à sécher l'implant ainsi revêtu, de préférence à une température comprise entre 35 °C et 80 °C.
